# EUROPEAN PATENT APPLICATION

(11) **EP 1 632 572 A1**
(43) Date of publication of application: **08.03.2006**
(21) Application number: 05019135.2
(22) Date of filing: 02.09.2005
(51) Int. Cl.: C12N 15/10

(54) **Method for separating and purifying nucleic acid**

(30) Priority: 03.09.2004 JP 2004257202; 01.09.2005 JP 2005253576
(71) Applicant: FUJI PHOTO FILM CO., LTD., Minami-Ashigara-shi, Kanagawa (JP)
(72) Inventor: Iwaki, Yoshihide, Asaka-shi, Saitama (JP); Mori, Toshihiro, Asaka-shi, Saitama (JP)
(74) Representative: Hiebl, Inge Elisabeth

(57) **Abstract**

Nucleic acid contained in a sample is highly efficiently recovered at a high recovery ratio by a method for separating and purifying nucleic acid using whole blood as the sample, which is a method for separating and purifying nucleic acid, comprising: preparing a sample solution containing nucleic acid; putting the sample solution containing nucleic acid in contact with a solid phase to allow nucleic acid to be adsorbed to the solid phase; putting a washing solution in contact with the solid phase to wash the solid phase at the state of nucleic acid adsorbed thereon; and putting a elution solution in contact with the solid phase to allow nucleic acid to be desorbed from the solid phase, wherein the step of preparing a sample solution containing nucleic acid comprises at least one selected from the group consisting of vortexing, mixing with inversion, and pipetting.

## Description

### 1. Field of the Invention

The invention relates to a method for separating and purifying nucleic acid. More specifically, the invention relates to a solid phase and a sample solution containing nucleic acid for use in a method for separating and purifying nucleic acid, and also relates to a method for separating and purifying nucleic acid using them.

### 2. Background Art

Nucleic acid is used in various forms in diverse fields. For example, it is required in the field of recombinant nucleic acid technology to use nucleic acid in the forms of probe, genomic nucleic acid and plasmid nucleic acid.

Even in the field of diagnosis, nucleic acid is used in various forms for various purposes. For example, nucleic acid probe is routinely used for detecting and diagnosing pathogens in humans. Additionally, nucleic acid is used for detecting genetic disorders and detecting substances contaminated in foods. For various purposes including the preparation of genetic map, cloning and gene expression via gene recombination, further, nucleic acid is routinely used for the identification of the position of a given nucleic acid and for the identification and isolation of nucleic acid.

In many cases, however, only a trace amount of nucleic acid can be obtained. The procedures for the isolation and purification thereof are laborious and time-consuming. Such laborious, time-consuming processes readily lead to the loss of nucleic acid, disadvantageously. In case of purifying nucleic acid from a sample obtained by culturing serum, urine and bacteria, additionally, contamination disadvantageously occurs, so that false positivity disadvantageously emerges.

As one of methods for solving the problems described above and separating and purifying nucleic acid highly efficiently in a simple manner, patent reference 1 (Japanese Patent Laid-open No. 2003/128691) discloses a method for separating and purifying nucleic acid, including allowing nucleic acid to be adsorbed to a solid phase comprising an organic polymer with hydroxyl group on the surface and then allowing the nucleic acid to be desorbed from the solid phase, individually using a solution for the adsorption of nucleic acid onto the solid phase and a solution for the desorption of nucleic acid from the solid phase.

Particularly when whole blood is used as a sample in carrying out such method for separating and purifying nucleic acid, however, the solution obtained from the sample is at a higher viscosity. Additionally because whole blood contains various cell components, simple addition of a surfactant, a chaotropic salt or a mixed solution thereof cannot disrupt such cell components sufficiently enough to lyse cell membrane to solubilize nucleic acid. Therefore, the amount of nucleic acid to be recovered by the nucleic acid separation and purification is smaller, disadvantageously. When the solid phase for the absorption and desorption of nucleic acid by the method for separating and purifying nucleic acid is in a membrane form, furthermore, the solid phase is so readily clogged, disadvantageously, that the processing efficiency is lowered.

### Summary of the Invention

It is an object of the invention to provide a method for separating and purifying nucleic acid including a step of allowing nucleic acid to be adsorbed to a solid phase, washing the solid phase at the state of nucleic acid adsorbed thereon, and a step of allowing nucleic acid to be desorbed from the solid phase, where nucleic acid contained in a sample can be recovered highly efficiently. It is an additional object of the invention to provide a method for separating and purifying nucleic acid, which allows nucleic acid to be recovered at a high yield and high processing efficiency, even when whole blood is used as such sample.

As the results of intensive research works, the present inventors found that nucleic acid could be recovered highly efficiently by vortexing, pipetting, mixing with inversion and the like in preparing a sample solution containing nucleic acid. The inventors also found that nucleic acid could be recovered highly efficiently by vortexing, pipetting, mixing with inversion and the like for highly efficiently mixing together a proteinase, a sample and a pretreating solution, to thereby disrupt cell components to lyse cell membrane and solubilize nucleic acid. The inventors further found that more efficient mixing could be done in a manner depending on the vortex velocity and the mixing order of the pretreating solution, to recover nucleic acid highly efficiently.

In accordance with the invention, the objects have been achieved with the following constitutions.
1. A method for separating and purifying nucleic acid, comprising:
   preparing a sample solution containing nucleic acid;
   putting the sample solution containing nucleic acid in contact with a solid phase to allow nucleic acid to be adsorbed to the solid phase;
   putting a washing solution in contact with the solid phase to wash the solid phase at the state of nucleic acid adsorbed thereon; and
   putting a elution solution in contact with the solid phase to allow nucleic acid to be desorbed from the solid phase,
   wherein the step of preparing a sample solution containing nucleic acid comprises at least one selected from the group consisting of vortexing, mixing with inversion, and pipetting.
2. A method for separating and purifying nucleic acid according to the item 1, wherein the step of preparing a sample solution containing nucleic acid comprises: adding a proteinase, a sample, and a pretreating solution containing at least one selected from the group consisting of chaotropic salts, surfactants, defoaming agents, nucleic acid stabilizers and buffers, in this order, or adding the pretreating solution, a sample and a proteinase, in this order; and subsequently carrying out at least one selected from the group consisting of vortexing, mixing with inversion, and pipetting.
3. A method for separating and purifying nucleic acid according to the item 1, wherein the step of preparing a sample solution containing nucleic acid comprises: adding a proteinase, a sample, and a pretreating solution containing at least one selected from the group consisting of chaotropic salts, surfactants, defoaming agents, nucleic acid stabilizers and buffers, in this order, or adding the pretreating solution, a sample and a proteinase, in this order, adding a water-soluble organic solvent; and carrying out at least one selected from the group consisting of vortexing, mixing with inversion, and pipetting.
4. A method for separating and purifying nucleic acid according to the item 1, wherein the step of preparing a sample solution containing nucleic acid comprises; adding a proteinase, a sample, and a pretreating solution containing at least one selected from the group consisting of chaotropic salts, surfactants, defoaming agents, nucleic acid stabilizers and buffers, in this order, or adding the pretreating solution, a sample and a proteinase, in this order; carrying out at least one selected from the group consisting of vortexing, mixing with inversion, and pipetting; and adding a water-soluble organic solvent.
5. A method for separating and purifying nucleic acid according to the item 1, wherein the step of preparing a sample solution containing nucleic acid comprises: adding a proteinase, a sample, and a pretreating solution containing at least one selected from the group consisting of chaotropic salts, surfactants, defoaming agents, nucleic acid stabilizers and buffers, in this order, or adding the pretreating solution, a sample and a proteinase, in this order; subsequently carrying out at least one selected from the group consisting of vortexing, mixing with inversion, and pipetting; adding a water-soluble organic solvent; and carrying out at least one selected from the group consisting of vortexing, mixing with inversion, and pipetting.
6. A method for separating and purifying nucleic acid according to any one of the items 1 to 5, wherein the sample solution containing nucleic acid is obtained by the preparation of whole blood as a sample.
7. A method for separating and purifying nucleic acid according to any one of the items 1 to 6, wherein the step of preparing a sample solution containing nucleic acid comprises vortexing at 2,000 rpm or more.
8. A method for separating and purifying nucleic acid according to any one of the items 1 to 6, wherein the step of preparing a sample solution containing nucleic acid comprises performing once or more of mixing with inversion or pipetting in combination with vortexing at less than 2,500 rpm.
9. A method for separating and purifying nucleic acid according to any one of the items 1 to 6, wherein the step of preparing a sample solution containing nucleic acid comprises performing once or more of mixing with inversion or pipetting in combination with vortexing at less than 2,000 rpm.
10. A method for separating and purifying nucleic acid according to any one of the items 3 to 9, wherein the water-soluble organic solvent includes at least one selected from the group consisting of methanol, ethanol, propanol and butanol.
11. A method for separating and purifying nucleic acid according to any one of the items 1 to 10, wherein the solid phase is in a membrane form.
12. A method for separating and purifying nucleic acid according to any one of the items 1 to 11, wherein the solid phase contains silica or a derivative thereof, diatomaceous earth, or alumina.
13. A method for separating and purifying nucleic acid according to any one of the items 1 to 12, wherein the solid phase contains an organic polymer.
14. A method for separating and purifying nucleic acid according to the item 13, wherein the organic polymer is an organic polymer having a polysaccharide structure.
15. A method for separating and purifying nucleic acid according to the item 13 or 14, wherein the organic polymer is acetylcellulose.
16. A method for separating and purifying nucleic acid according to the item 13 or 14, wherein the organic polymer is an organic polymer prepared by a process of saponifying acetylcellulose or a mixture of acetylcellulose having different acetyl values.
17. A method for separating and purifying nucleic acid according to the item 13 or 14, wherein the organic polymer is regenerated cellulose,

By the method for separating and purifying nucleic acid including a step of allowing nucleic acid to be adsorbed to a solid phase, a step of washing the solid phase at the state of nucleic acid adsorbed thereon, and a step of allowing nucleic acid to be desorbed from the solid phase, nucleic acid contained in a sample can be recovered highly efficiently. Even when whole blood is used as a sample, nucleic acid can be recovered at high processing efficiency and a high yield.

### DETAILED DESCRIPTION OF THE INVENTION

The method for separating and purifying nucleic acid in accordance with the invention includes at least
(1) a step of putting a sample solution containing nucleic acid in contact with a solid phase to allow nucleic acid to be adsorbed to the solid phase (sometimes referred to as "adsorption step"),
(2) a step of putting a washing solution in contact with the solid phase to wash the solid phase at the state of nucleic acid adsorbed thereon (sometimes referred to as "washing step"), and
(3) a step of putting a elution solution in contact with the solid phase to allow nucleic acid to be desorbed from the solid phase (sometimes referred to as "recovery step").

In accordance with the invention, specifically, putting a sample solution containing nucleic acid in contact with a solid phase enables the adsorption of nucleic acid in the sample solution onto the solid phase; the solid phase is washed; and then, the nucleic acid adsorbed to the solid phase is desorbed from the solid phase, using a elution solution.

In accordance with the invention, furthermore, the sample solution containing nucleic acid can be obtained by the step of preparing a sample solution containing nucleic acid as described below.

### <Step of preparing sample solution containing nucleic acid>

In accordance with the invention, the step of preparing a sample solution containing nucleic acid (sometimes referred to as sample solution preparation step hereinbelow) includes at least one of vortexing, mixing with inversion and pipetting. Preferably, the step of preparing a sample solution containing nucleic acid includes adding a proteinase, a sample, a pretreating solution containing at least one selected from the group consisting of chaotropic salts, surfactants, defoaming agents, nucleic acid stabilizers and buffers in this order and subsequently carrying out at least one selected from the group consisting of vortexing, mixing with inversion and pipetting.

### (Vortexing, mixing with inversion and pipetting)

In accordance with the invention, "vortex" and "vortexing" mean for example procedures mixing with vortex mixer. Any vortexing form may be satisfactory with no specific limitation, as long as vortexing procedures can be done with the vortexing form. For example, vortex mixer is preferably used. Commercially available vortex mixers can be used as such.

Vortexing is preferably done at 2,000 rpm or more. The solution can sufficiently be mixed together by vortexing at 2,000 rpm or more, preferably, so that cell components contained in the sample, particularly in whole blood can sufficiently be disrupted, to lyse cell membrane to readily solubilize nucleic acid.

Even in case that a membrane form is used as the solid phase, preferably, clogging can be prevented, so that the processing efficiency can be elevated.

Generally, vortexing is routinely done at about 600 rpm.

When vortexing is done at less than 2,000 rpm, vortexing is preferably done in combination with pipetting or mixing with inversion. The combination of pipetting or mixing with inversion produces the same effect as in the case of vortexing at 2,000 rpm or more. Pipetting or mixing with inversion is done preferably once or more, more preferably three times or more, and still more preferably five times or more.

Pipetting can be done using commercially available micropipettes and the like. Using a micropipette, the solution containing a sample solution is aspirated or ejected repeatedly in a manner depending on the volume of the solution.

Mixing with inversion can be done by shaking a container placing therein a solution containing a sample by hands several times or using a commercially available shaker. Any container shape is satisfactory, as long as the container can be shaken.

The step of preparing a sample solution containing nucleic acid includes adding a proteinase, a sample, a pretreating solution containing at least one selected from the group consisting of chaotropic salts, surfactants, defoaming agents, nucleic acid stabilizers and buffers in this order and then carrying out at least one selected from the group consisting of vortexing, mixing with inversion and pipetting. The addition of a proteinase, a sample and the pretreating solution in this order preferably promotes the solubilization of nucleic acid. In case that the pretreating solution contains chaotropic salts such as guanidine hydrochloride, in particular, the proteinase may sometimes be inactivated with the pretreating solution. Thus, the order described above is preferable. The order of the addition thereof may satisfactorily be an order of the pretreating solution, a sample and a proteinase. By adding them in the order, the amount and efficiency of the recovered nucleic acid are improved, so that a nucleic acid-containing sample required therefor can preferably be reduced to a very trace amount. Additionally, the procedures therefor can be finished in a very short time, preferably.

Further, the step of preparing a sample solution containing nucleic acid preferably includes adding a proteinase, a sample, a pretreating solution containing at least one selected from the group consisting of chaotropic salts, surfactants, defoaming agents, nucleic acid stabilizers and buffers in this order, subsequently carrying out at least one selected from the group consisting of vortexing, mixing with inversion and pipetting and additionally adding a water-soluble organic solvent or the step preferably includes adding the pretreating solution, a sample and a proteinase in this order, additionally adding a water-soluble organic solvent and subsequently carrying out at least one selected from the group consisting of vortexing, mixing with inversion and pipetting. Due to the same reason as described above, the order of adding a proteinase, a sample and the pretreating solution may be an order of adding the pretreating solution, a sample and a proteinase.

Still further, the step of preparing a sample solution containing nucleic acid preferably includes adding a proteinase, a sample, a pretreating solution containing at least one selected from the group consisting of chaatropic salts, surfactants, defoaming agents, nucleic acid stabilizers and buffers in this order, carrying out at least one selected from the group consisting of vortexing, mixing with inversion and pipetting, adding a water-soluble organic solvent and carrying out at least one selected from the group consisting of vortexing, mixing with inversion and pipetting. Due to the same reason as described above, the order of adding a proteinase, a sample and the pretreating solution may be an order of adding the pretreating solution, a sample and a proteinase.

At the step of preparing a sample solution as described above, incubation is particularly preferably done at a temperature of 25 to 70 °C and is most preferably done at the optimal temperature of a proteinase among others. The timing of incubation is after adding the pretreating solution and carrying out at least one selected from the group consisting of vortexing, mixing with inversion and pipetting.

### (Sample)

Any sample containing nucleic acid may be used as the sample in accordance with the invention, with no specific limitation. In the diagnostic field, for example, the subjects are blood-derived components such as collected whole blood, plasma or serum, body fluids such as urine, feces, seminal fluid and saliva, or biological materials such as plants (or parts thereof), animals (or parts thereof), bacteria and viruses. As such samples, these are used as they are or these are used as lysed products or homogenates. Preferably, the sample is collected whole blood.

"Sample" means an appropriate sample containing nucleic acid. One or two or more types of nucleic acid may be contained in the sample solution. The lengths of the individual nucleic acids to be subjected to the method for separating and purifying nucleic acid are not specifically limited. For example, nucleic acid of an appropriate length of several bp to several Mbp is exemplified. From the manipulation standpoint, generally, the length of nucleic acid is preferably several bp to several hundreds kbp.

In accordance with the invention, "nucleic acid" may be any of DNA or RNA, single-stranded or double-stranded. The molecular weight thereof is not specifically limited.

When the subject sample is whole blood, preferably, leukocyte and nuclear membrane are lysed. The lysis of leukocyte and the lysis of nuclear membrane are done for efficiently solubilizing nucleic acid as an extraction subject.

More preferably, erythrocyte and various proteins are eliminated. The elimination of erythrocyte and various proteins is preferable because it effectively prevents the non-specific adsorption thereof onto the solid phase and the clogging of a porous membrane when it is used as the solid phase.

Specifically, for example, proteinase K, a whole blood sample, and a pretreating solution in mixture with guanidine hydrochloride, surfactants and the like are added in this order, followed by vortexing at 2,500 rpm or more and incubation at 60 °C for 10 minutes.

As such protease, at least one protease selected from among serine protease, cysteine protease, metal protease, etc. can preferably be used. Also, a mixture of plural kinds of proteases may preferably be used.

Serine protease is not particularly limited and, for example, protease K can preferably be used. Cysteine protease is not particularly limited and, for example, papain and cathepsin may preferably be used.

Metal protease is not particularly limited and, for example, carboxypeptidase may preferably be used.

The protease can be used, upon addition, in an amount of preferably from 0.0003 IU to 3 IU, more preferably from 0.003 IU to 0.3 IU, per ml of the whole reaction system.

Also, as the protease, a protease not containing nuclease can preferably be used. Also, a protease containing a stabilizing agent can preferably be used. As the stabilizing agent, a metal ion can preferably be used. Specifically, magnesium ion is preferable, and can be added in the form of, for example, magnesium chloride. Incorporation of a stabilizing agent for a protease enables one to reduce the amount of protease necessary for recovery of nucleic acids to a slight amount, which serves to reduce the cost required for recovery of nucleic acids. The amount of the stabilizing agent for protease is preferably from 1 to 1000 mmol/L, more preferably from 10 to 100 mmol/L, based on the whole amount of the reaction system.

### {Pretreating solution}

As described above, the pretreating solution to be used in accordance with the invention contains at least one selected from the group consisting of chaotropic salts, surfactants, defoaming agents, nucleic acid stabilizers and buffers.

### (Chaotropic salts)

As the chaotropic salts, for example, guanidine salts, sodium isothioate, sodium iodide and potassium iodide may be used. Among them, guanidine salts are preferable. The guanidine salts include guanidine hydrochloride, guanidine isothiocyanate, and guanidine thiocyanate. Guanidine hydrochloride is preferable among them. These salts may be used singly or in combination of plural such salts. The concentration of the chaotropic salts in the pretreating solution is preferably 0.5 mol/L or more, more preferably 0.5 mol/L to 4 mol/L, still more preferably 1 mol/L to 3 mol/L.

Instead of the chaotropic salts, urine may be used as a chaotropic substance.

Surfactants, for example, include a nonionic surfactant, a cationic surfactant, an anionic surfactant, an amphoteric surfactant.

In the invention, the nonionic surfactant and the cationic surfactant can be preferably used.

Nonionic surfactants include a polyoxyethylene alkyl phenyl ether-based surfactant, a polyoxyethylene alkyl ether-based surfactant, and fatty acid alkanolamide, and the preferable one is a polyoxyethylene alkyl ether-based surfactant. Among the polyoxyethylene (POE) alkyl ether surfactant, POE decyl ether, POE lauryl ether, POE tridecyl ether, POE alkylenedecyl ether, POE sorbitan monolaurate, POE sorbitan monooleate, POE sorbitan monostearate, tetraoleic polyoxyethylene sorbit, POE alkyl amine, and POE acetylene glycol are more preferred.

Cationic surfactants include cetyl trimethyl ammonium bromide, dodecyl trimethyl ammonium chloride, tetradecyl trimethyl ammonium chloride, cetyl pyridinium chloride.

These surfactants can be used alone or in combinations of two or more. The concentration of the surfactant in the nucleic acid-solubilizing reagent is preferably from 0.1 to 20% by weight.

As the defoaming agent, a silicon-based defoaming agent (e.g., silicon oil, dimethyl polysiloxane, silicon einersion, denatured polysiloxane, silicon compound, etc.), alcohol-based defoaming agent (e.g., acetylene glycol, heptanol, ethyl exanol, superhigh grade alcohol, polyoxy alkylene glycol, etc.), ether-based defoaming agent (e,g., heptyl cellosolve, nonyl cellosolve-3-heptylcorbitol, etc.), fatty oil-based defoaming agent (e.g., animal and plant fat, etc.), fatty acid-based defoaming agent (e.g., stearic acid, oleic acid, palmitic acid, etc.), metallic soap-based defoaming agent (e.g., aluminum stearate, calcium stearate, etc.), fatty acid ester-based defoaming agent (e.g., a natural wax, tributyl phosphate, etc.), phosphate ester-based defoaming agent (e.g., sodium octyl phosphate, etc.), amine-based defoaming agent (e.g., diamyl amine, etc.), amide-based defoaming agent (e.g., amide stearate, etc.), and other defoaming agents (e.g., ferric sulfate, bauxite, etc.) can be exemplified. These defoaming agent can be used alone or in combinations of two or more. Two compounds combined from silicon-based and alcohol-based defoaming agents are especially preferred.

The concentration of a defoaming agent in nucleic acid-solubilizing reagent is preferably in a range of 0.05 to 20% by weight.

As the nucleic acid stabilizing agent, one having a reaction to inactivate a nuclease activity can be exemplified. Depending on a test sample, there are cases where nuclease, which degrades nucleic acid, is comprised thereto so that when nucleic acid is homogenized, nuclease reacts with nucleic acid, so as to result in a remarkable reduction of a yield amount. For the purpose of avoiding this, a stabilizing agent having a function to inactivate nuclease can be coexisted in a nucleic acid-solubilizing solution. As a result, improvements in a recovering yield and a recovering efficiency of nucleic acid lead to the minimization and acceleration of a test sample.

As the nucleic acid stabilizing agent having functions to inactivate the nuclease activity, a compound used routinely as a reducing agent can be used. Examples of reducing agents include hydrogenated compounds such as a hydrogen atom, hydrogen iodide, hydrogen sulfide, aluminum lithium hydride, and sodium borohydride; a highly electropositive metal such as alkaline metal, magnesium, calcium, aluminum, and zinc, or their amalgam, organic oxides such as aldhyde-based, sugar-based, formic acid, and oxalic acid; and mercapto compounds. Among these, the mercapto compounds are preferable. Examples of mercapto compounds include N-acetyl cysteine, mercapto ethanol, and alkyl mercaptane or the like. The mercapto compounds can be used alone or in combinations of two or more.

The concentration of the nucleic acid stabilizing agent in the nucleic acid-solubilizing reagent is preferably from 0.1 to 20% by weight, and more preferably from 0.3 to 15% by weight. The concentration of the mercapto compounds in the nucleic acid-solubilizing reagent is preferably from 0.1 to 10% by weight, and more preferably from 0.5 to 5% by weight.

Further, chelating agents may be used as the nucleic acid stabilizers with an action to inactivate nuclease. The chelating agents include for example ethylenediaminetetraacetic acid (EDTA), nitrilotriacetic acid (NTA) and EGTA. The chelating agents may be used singly or in combination of plural such agents. The chelating agents can be used at a concentration of preferably 1 mmol/L to 1 mol/L, more preferably 5 mmol/L to 100 mmol/L in the pretreating solution.

### (Buffers)

The buffers include pH buffers for routine use.

Preferably, the buffers include pH buffers for routine use at biochemical tests. Such buffers include buffers comprising citrate salts, phosphate salts or acetate salts, Tris-HCl, TE (Tris-HCl/EDTA), TBE (Tris-borate/EDTA), TAE (Tris-acetate/EDTA) and the Good's buffer. The Good's buffer includes MES (2-morpholinoethanesulfonic acid), Bis-Tris [bis(2-hydroxyethyl)iminotris(hydroxymethyl)methane], HEPES (2-[4-(2-hydroxyethyl)-1-piperazinyl]ethanesulfonic acid), PIPES [piperaxine-1,4-bis(2-ethanesulfonic acid)], ACES [N-(2-acetamino)-2-aminoethanesulfonic acid], CAPS (N-cyclohex-yl-3-aminopropanesulfonic acid), and TES [N -tris(hydroxymethyl)methy 1-2-aminoethanesulfonic acid].

These buffers are preferably at a concentration of 1 to 300 mmol/L in the pretreating solution.

The pretreating solution is preferably supplied in a dry state, namely in the form of a pretreating agent. Additionally, a container preliminarily containing a proteinase at a dry state such as freeze-dried state may also be used. Using the pretreating agent and/or the container preliminarily containing the proteinase at dry state, a sample solution containing nucleic acid may be obtained.

In case that a sample solution containing nucleic acid is to be obtained by the method, preferably, the storage stability of the pretreating agent and the proteinase at dry state is so high that the procedures can be done in a simple manner without any change of the yield of nucleic acid.

From the standpoint of improving the solubility of the compounds contained in the pretreating solution, a water-soluble organic solvent may satisfactorily be added to the pretreating solution. The water-soluble organic solvent includes for example alcohols, acetone, acetonitrile, and dimethylformamide. Among them, alcohols are preferable. The alcohols are any of primary alcohol, secondary alcohol, and tertiary alcohol. Specifically, the alcohols include for example methanol, ethanol, propanol and isomers thereof, and butanol and isomers thereof. Among them, ethanol is particularly preferable. These water-soluble organic solvents may be used singly or in combination of plural such organic solvents. The pretreating solution is preferably prepared to 1 -20 % by mass as the concentration of the water-soluble organic solvent in a sample solution containing nucleic acid.

### <Water-soluble organic solvent and adsorption step>

The further addition of the water-soluble organic solvent after the addition of a proteinase, a sample and the pretreating solution at the step of preparing the sample solution containing nucleic acid as described above is preferable since nucleic acid in the sample solution can be effectively adsorbed to a solid phase by adding the water-soluble organic solvent to a solution of nucleic acid solubilized in dispersion to put the nucleic acid in contact with the solid phase. Further, the presence of salts in the resulting sample solution containing nucleic acid is preferable since the solubilized nucleic acid can more effectively be adsorbed to the solid phase. After the addition of a proteinase, a sample and the pretreating solution, at least one selected form vortexing, mixing with inversion and pipetting may satisfactorily be done, followed by the addition of the water-soluble organic solvent. Otherwise, at least one selected from the group consisting of vortexing, mixing with inversion and pipetting may satisfactorily be done after the addition of a proteinase, a sample and the pretreating solution and the addition of a water-soluble organic solvent. Further, at least one selected form vortexing, mixing with inversion and pipetting may be done after the addition of a proteinase, a sample and the pretreating solution. Even after the addition of a water-soluble organic solvent, additionally, at least one selected form vortexing, mixing with inversion and pipetting may be done.

The presence of a water-soluble organic solvent and salts permits the disruption of the hydrated structure of water molecules existing around nucleic acid to solubilize nucleic acid in unstable state. When the nucleic acid in that state is put in contact with a solid phase, the polar groups on the surface of the nucleic acid interact with the polar groups of the solid phase surface. Thus, it is understood that the nucleic acid is adsorbed to the solid phase surface. When an organic polymer with hydroxyl group on the surface is used as the solid phase, preferably, the adsorption occurs greatly. According to the method of the invention, the addition of a water-soluble organic solvent together with the presence of salts in the resulting sample solution containing nucleic acid can make nucleic acid unstable, preferably, as described above.

The water-soluble organic solvent includes for example alcohols, acetone, acetonitrile, and dimethylformamide. Among them, alcohols are preferable. The alcohols are any of primary alcohol, secondary alcohol, and tertiary alcohol. Specifically, the alcohols include for example methanol, ethanol, propanol and isomers thereof, and butanol and isomers thereof. Among them, ethanol is more preferably used. These water-soluble organic solvents may be used singly or in combination of plural such organic solvents.

The final concentration of such water-soluble organic solvent in the sample solution containing nucleic acid is preferably 5 to 90 % by mass. It is particularly preferable that the concentration of ethanol added is as high as possible within the range without any generation of aggregates. More preferably, the final concentration thereof is 20 % by mass to 60 % by mass.

Salts preferably existing in the resulting sample solution containing nucleic acid include for example various chaotropic substances (guanidium salts, sodium iodide, and sodium perchlorate), sodium chloride, potassium chloride, ammonium chloride, sodium bromide, potassium bromide, calcium bromide and ammonium bromide. Guanidium salts are particularly preferable because the salts have effects on both the lysis of cell membrane and the solubilization of nucleic acid.

The pH of the resulting sample solution is preferably pH 3 to 10, more preferably pH 4 to 9, still more preferably pH 5 to 8.

The resulting sample solution containing nucleic acid is preferably at a surface tension within a range of 0.05 J/m² or less, a viscosity within a range of 1 to 10,000 mPa, and a specific gravity within a range of 0.8 to 1.2. When the sample solution is adjusted to the ranges, the solution remaining after the adsorption of nucleic acid via the contact of the sample solution containing nucleic acid with the solid phase at the adsorption step is readily removed at the washing step.

### [Solid phase]

As the solid phase, any material capable of adsorbing nucleic acid thereon may be used with no specific limitation. There may be used for example solid phases comprising an organic polymer with hydroxyl group on the surface, or solid phases comprising silicon dioxide, silica polymer or magnesium silicate, or solid phases comprising silica or derivatives thereof, diatomaceous earth or alumina. Preferably, solid phases comprising an organic polymer with a polysaccharide structure may be used. More preferable are solid phases adsorbing nucleic acid thereon via an interaction without any substantial involvement of ionic bond. This means "no ionization" under the solid phase conditions for use. It is inferred that the change of the polarity in the environment will allow nucleic acid and the solid phases to be drawn together. In such manner, nucleic acid can be isolated and purified with such great separation profile and at a high washing efficiency. Solid phases adsorbing nucleic acid thereon via an interaction without any substantial involvement of ionic bond include for example solid phases with hydrophilic groups. It is inferred that via the change of the polarity in the environment, the hydrophilic groups of nucleic acid and the hydrophilic groups of the solid phase will be drawn together.

The hydrophilic group means a polar group (atoms) capable of exerting an interaction with water, and includes all groups (atoms) participating in adsorption of nucleic acid. As the hydrophilic group, those which exhibit about a middle level of interaction with water (see, "group having not so strong hydrophilicity" in the item of "hydrophilic group" described in Kagaku Dai-jiten, published by Kyoritsu Shuppan) are preferred, and examples thereof include a hydroxyl group, a carboxyl group, a cyano group and a hydroxyethyl group, with a hydroxyl group being preferred.

Here, the term "solid phase having a hydrophilic group" means a solid phase wherein the material constituting the solid phase itself has the hydrophilic group, or a solid phase obtained by treating or coating a solid phase-constituting material in order to introduce the hydrophilic group into the solid phase. The solid phase-constituting material may be an organic or inorganic material. For example, there may be used a solid phase wherein the solid phase-constituting material itself is an organic material having a hydrophilic group, a solid phase which is obtained by treating a solid phase made of a hydrophilic group-free organic material so as to introduce the hydrophilic group thereinto, a solid phase obtained by coating a solid phase made of a hydrophilic group-free organic material with a material having a hydrophilic group to thereby introduce the hydrophilic group, a solid phase wherein the solid phase-constituting material itself is an inorganic material having a hydrophilic group, a solid phase which is obtained by treating a solid phase made of a hydrophilic group-free inorganic material so as to introduce the hydrophilic group thereinto, and a solid phase obtained by coating a solid phase made of a hydrophilic group-free inorganic material with a material having a hydrophilic group to thereby introduce the hydrophilic group. In view of processing ease, it is preferable to use an organic material such as an organic polymer as the material for constituting the solid phase.

With regard to the solid phase of an organic material having hydroxyl group which is able to be used in the present invention, its examples includes solid phase formed by polyhydroxyethylacrylic acid, polyhydroxyethylmethacrylic acid, polyvinyl alcohol, polyvinylpyrrolidone, polyacrylic acid, polymethacrylic acid and polysaccharide such as polyoxyethylene and acetylcellulose acetylcellulose mixture having different acetyl values and solid phase of an organic material having a polysaccharide structure is able to be used particularly preferably.

As the organic material having a polysaccharide structure, cellulose, hemicellulose, dextran, amylase, amylopectin, starch, glycogen, pullulan, mannan, glucomannan, lichenan, isolichenan, laminaran, carrageenan, xylan, fructan, alginic acid, hyaluronic acid, chondroitin, chitin and chitosan can preferably be used. However, these are not limitative, and any organic material having a polysaccharide structure or its derivative may be used. Also, an ester derivative of any of these polysaccharides can preferably be used. Further, a saponification product of the ester derivative of any of these polysaccharides can preferably be used.

As the ester of the ester derivative of any of the above-mentioned polysaccharides, one or more members selected from among carboxylates, nitrates, sulfates, sulfonates, phosphates, phosphonates and pyrophosphates are preferably selected. Also, saponification products of the carboxylates, nitrates, sulfates, sulfonates, phosphates, phosphonates and pyrophosphates can more preferably be used.

As the carboxylates of any of the above-mentioned polysaccharides, one or more members selected from among alkylcarbonyl esters, alkenylcarbonyl esters, aromatic carbonyl esters and aralkylcarbonyl esters are preferably selected. Also, saponification products of the alkylcarbonyl esters, alkenylcarbonyl esters, aromatic carbonyl esters and aralkylcarbonyl esters of any of the above-mentioned polysaccharides can more preferably be used.

As the ester group of the alkylcarbonyl esters of any of the above-mentioned polysaccharides, one or more members selected from among an acetyl group, a propionyl group, a butyroyl group, a valeryl group, a heptanoyl group, an octanoyl group, a decanoyl group, a dodecanoyl group, a tridecanoyl group, a hexadecanoyl group and an octadecanoyl group are preferably selected. Also, saponification products of any of the above-mentioned polysaccharides having one or more ester groups selected from among an acetyl group, a propionyl group, a butyloyl group, a valeryl group, a heptanoyl group, an octanoyl group, a decanoyl group, a dodecanoyl group, a tridecanoyl group, a hexadecanoyl group and an octadecanoyl group can more preferably be used.

As the ester group of the alkenylcarbonyl esters of any of the above-mentioned polysaccharides, one or more of an acryl group and a methacryl group are preferably selected. Also, saponification products of any of the above-mentioned polysaccharides having ester groups of one or more of an acyl group and a methacryl group can more preferably be used.

As the ester group of the aromatic carbonyl esters of any of the above-mentioned polysaccharides, one or more of a benzoyl group and a naphthaloyl group are preferably selected. Also, saponification products of any of the above-mentioned polysaccharides having ester groups of one or more of a benzoyl group and a naphthaloyl group can more preferably be used.

As the nitrates of any of the polysaccharides, nitrocellulose, nitrohemicellulose, nitrodextran, nitroagarose, nitrodextrin, nitroamylase, nitroamylopectin, nitroglycogen, nitropullulan, nitromannan, nitroglucomannan, nitrolichenan, nitroisolichenan, nitrolaminaran, nitrocarrageenan, nitroxylan, nitrofructan, nitroalginic acid, nitrohyaluronic acid, nitrochondroitin, nitrochitin and nitrochitosan can preferably be used.

Also, saponification products of nitrocellulose, nitrohemicellulose, nitrodextran, nitroagarose, nitrodextrin, nitroamylase, nitroamylopectin, nitroglycogen, nitropullulan, nitromannan, nitroglucomannan, nitrolichenan, nitroisolichenan, nitrolaminaran, nitrocarrageenan, nitroxylan, nitrofructan, nitroalginic acid, nitrohyaluronic acid, nitro chondroitin, nitrochitin and nitrochitosan can more preferably be used.

As the sulfates of any of the polysaccharides, cellulose sulfate, hemicellulose sulfate, dextran sulfate, agarose sulfate, dextrin sulfate, amylase sulfate, amylopectin sulfate, glycogen sulfate, pullulan sulfate, mannan sulfate, glucomannan sulfate, lichenan sulfate, isolichenan sulfate, laminaran sulfate, carrageenan sulfate, xylan sulfate, fructan sulfate, alginic acid sulfate, hyaluronic acid sulfate, chondroitin sulfate, chitin sulfate and chitosan sulfate can preferably be used.

Also, saponification products of cellulose sulfate, hemicellulose sulfate, dextran sulfate, agarose sulfate, dextrin sulfate, amylase sulfate, amylopectin sulfate, glycogen sulfate, pullulan sulfate, mannan sulfate, glucomannan sulfate, lichenan sulfate, isolichenan sulfate, laminaran sulfate, carrageenan sulfate, xylan sulfate, fructan sulfate, alginic acid sulfate, hyaluronic acid sulfate, chondroitin sulfate, chitin sulfate and chitosan sulfate can more preferably be used.

As the sulfonates of any of the aforementioned polysaccharides, one or more members selected from among alkyl sulfonates, alkenyl sulfonates, aromatic sulfonates and aralkyl sulfonates are preferably selected. Also, saponification products of alkyl sulfonates, alkenyl sulfonates, aromatic sulfonates and aralkyl sulfonates of any of the above-mentioned polysaccharides can more preferably be used.

As the phosphates of any of the aforementioned polysaccharides, cellulose phosphate, hemicellulose phosphate, dextran phosphate, agarose phosphate, dextrin phosphate, amylase phosphate, amylopectin phosphate, glycogen phosphate, pullulan phosphate, mannan phosphate, glucomannan phosphate, lichenan phosphate, isolichenan phosphate, laminaran phosphate, carrageenan phosphate, xylan phosphate, fructan phosphate, alginic acid phosphate, hyaluronic acid phosphate, chondroitin phosphate, chitin phosphate and chitosan phosphate can preferably be used.

Also, saponification products of cellulose phosphate, hemicellulose phosphate, dextran phosphate, agarose phosphate, dextrin phosphate, amylase phosphate, amylopectin phosphate, glycogen phosphate, pullulan phosphate, mannan phosphate, glucomannan phosphate, lichenan phosphate, isolichenan phosphate, laminaran phosphate, carrageenan phosphate, xylan phosphate, fructan phosphate, alginic acid phosphate, hyaluronic acid phosphate, chondroitin phosphate, chitin phosphate and chitosan phosphate can more preferably be used.

As the phosphonates of any of the aforementioned polysaccharides, cellulose phosphonate, hemicellulose phosonphate, dextran phosphonate, agarose phosphonate, dextrin phosphonate, amylase phosphonate, amylopectin phosonphate, glycogen phosphonate, pullulan phosphonate, mannan phosphonate, glucomannan phosphonate, lichenan phosphonate, isolichenan phosphonate, laminaran phosphonate, carrageenan phosphonate, xylan phosphonate, fructan phosphonate, alginic acid phosphonate, hyaluronic acid phosphonate, chondroitin phosphonate, chitin phosphonate and chitosan phosphonate can preferably be used.

Also, saponification products of cellulose phosphonate, hemicellulose phosphonate, dextran phosphonate, agarose phosphonate, dextrin phosphonate, amylase phosphonate, amylopectin phosphonate, glycogen phosphonate, pullulan phosphonate, mannan phosphonate, glucomannan phosphonate, lichenan phosphonate, isolichenan phosphonate, laminaran phosphonate, carrageenan phosphonate, xylan phosphonate, fructan phosphonate, alginic acid phosphonate, hyaluronic acid phosphonate, chondroitin phosphonate, chitin phosphonate and chitosan phosphonate can more preferably be used.

As the pyrophosphates of any of the aforementioned polysaccharides, cellulose pyrophosphate, hemicellulose pyrophosphate, dextran pyrophosphate, agarose pyrophosphate, dextrin pyrophosphate, amylase pyrophosphate, amylopectin pyrophosphate, glycogen pyrophosphate, pullulan pyrophosphate, mannan pyrophosphate, glucomannan pyrophosphate, lichenan pyrophosphate, isolichenan pyrophosphate, laminaran pyrophosphate, carrageenan pyrophosphate, xylan pyrophosphate, fructan pyrophosphate, alginic acid pyrophosphate, hyaluronic acid pyrophosphate, chondroitin pyrophosphate, chitin pyrophosphate and chitosan pyrophosphate can preferably be used.

Also, saponification products of cellulose pyrpphosphate, hemicellulose pyrophosphate, dextran pyrophosphate, agarose pyrophosphate, dextrin pyrophosphate, amylase pyrophosphate, amylopectin pyrophosphate, glycogen pyrophosphate, pullulan pyrophosphate, mannan pyrophosphate, glucomannan pyrophosphate, lichenan pyrophosphate, isolichenan pyrophosphate, laminaran pyrophosphate, carrageenan pyrophosphate, xylan pyrophosphate, fructan pyrophosphate, alginic acid pyrophosphate, hyaluronic acid pyrophosphate, chondroitin pyrophosphate, chitin pyrophosphate and chitosan pyrophosphate can more preferably be used.

As the ether derivatives of any of the aforementioned polysaccharides, methyl cellulose, ethyl cellulose, carboxymethyl cellulose, carboxyethyl cellulose, carboxyethyl-carbamoylethyl cellulose, hydroxymethyl cellulose, hydroxyethyl cellulose, hydroxypropyl cellulose, hydroxypropylmethyl cellulose, hydroxyethylmethyl cellulose, cyanoethyl cellulose and carbamoyethyl cellulose can be used, though the ether derivatives not being limited thereto. It is preferable to use hydroxymethyl cellulose or hydroxyethyl cellulose.

With regard to the particularly preferred solid phase of cellulose ester derivative, a solid phase of an organic macromolecular substance comprising acetylcelluloses having different acetyl values may be listed. With regard to a mixture of acetylcelluloses having different acetyl values, a mixture of triacetylcellulose and diacetylcellulose, a mixture of triacetylcellulose and minoacetylcellulose, a mixture of triacetylcellulose and diacetylcellulose and a mixture of diacetylcellulose and monoacetylcellulose may be preferably used. A mixture of triacetylcellulose and diacetylcellulose is used particularly preferably. It is preferred that a mixing ratio (ratio by weight) of a mixture of triacetylcellulose and diacetylcellulose is 99:1 to 1:99 and, more preferably, 90:10 to 50:50.

An example of porous membrane of an organic material having a polysaccharide structure is a surface saponified product of acetylcellulose mentioned in Japanese Patent Laid-Open No. 2003/128,691. The surface-saponified product of acetylcellulose is a product where a mixture of acetylcelluloses having different acetyl values is subjected to a saponifying treatment and preferably used ones thereof are a saponified product of a mixture of triacetylcellulose and diacetylcellulose, a saponified product of a mixture of triacetylcellulose, diacetylcellulose and monoacetylcellulose and a saponified product of a mixture of diacetylcellulose and monoacetylcellulose. It is preferred that a mixing ratio (ratio by weight) of a mixture of triacetylcellulose and diacetylcellulose is 99:1 to 1:99. It is more preferred that the mixing ratio of a mixing ratio of triacetylcellulose and diacetylcellulose is 90:10 to 50:50. In that case, amount (density) of hydroxyl groups on the surfaced of solid phase may be able to be controlled by the degree of oxidizing treatment (saponifying rate). In order to enhance the separating efficiency of nucleic acid, the more the amount (density) of hydroxyl groups, the better. For example, in the case of acetylcellulose such as triacetylcellulose, saponifying rate (surface saponifying rate) is preferably about 5% or more and, more preferably, it is 10% or more. In order to make the surface area of the organic macromolecular substance having hydroxyl groups large, it is preferred that porous membrane of acetylcellulose is subjected to a saponifying treatment. In that case, when a solid phase where surface and back are symmetric is used, there is an advantage that production is possible without discrimination of surface and back of the membrane while, when a solid phase where surface and back are asymmetric is used, there is an advantage that risk of clogging of pores can be reduced whereby that is preferably used.

So as to obtain the saponified product, saponification process is done. Herein, the term saponification process means putting an organic material with ester group in contact with a solution for saponification processing (for example, aqueous sodium hydroxide solution). In such manner, the part in contact with the solution for saponification processing, namely the surface of an organic material, is saponified. In case of acetylcellulose, the part in contact with the solution for saponification processing is prepared into regenerated cellulose with hydroxyl group introduced therein. The regenerated cellulose thus prepared differs from the original intact cellulose in terms of crystal state and the like. In accordance with the invention, a solid phase containing regenerated cellulose is particularly preferably used as the solid phase.

So as to modify the saponification ratio, additionally, the concentration of sodium hydroxide can be varied for the saponification process. The saponification ratio is readily measured by NMR (the saponification ratio is determined for example by the reduction of the peak of carbonyl group).

A method for introducing a hydrophilic group to a solid phase comprising organic material not having a hydrophilic group is to bond a graft polymer chain having a hydrophilic group in inner polymer strand or a side chain to a solid phase.

A method for bonding a graft polymer chain to an organic material of a solid phase include two methods such as a method for chemically bonding a solid phase with graft polymer chain, and a method for polymerizing a compound having a double bond capable of polymerization using a solid phase as a starter to form graft polymer chain.

Firstly, in the method in which the solid phase and graft polymer chain are chemically bonded, a polymer having a functional group capable of reacting with the solid phase in the terminus or side chain of the polymer is used, and they are grafted through a chemical reaction of this functional group with a functional group of the solid phase. The functional group capable of reacting with the solid phase is not particularly limited with the proviso that it can react with a functional group of the solid phase, and its examples include a silane coupling group such as alkoxysilane, isocyanate group, amino group, hydroxyl group, carboxyl group, sulfonate group, phosphate group, epoxy group, allyl group, methacryloyl group, acryloyl group and the like.

The method in which a compound having a polymerizable double bond is made into a graft polymer chain by polymerizing it using as the starting point is generally called surface graft polymerization. The surface graft polymerization method means a method in which an active species is provided on the base material surface by plasma irradiation, light irradiation, heating or the like method, and a polymerizable compound having double bond arranged in contact with a solid phase is linked to the porous membrane by polymerization.

It is necessary that the compound useful for forming a graft polymer chain linked to the base material has both of two characteristics of having a polymerizable double bond and having a hydrophilic group which is concerned in the adsorption of nucleic acid. As such a compound, any one of the polymers, oligomers and monomers having a hydrophilic group can be used with the proviso that it has a double bond in the molecule. Particularly useful compound is a monomer having a hydrophilic group.

As illustrative examples of the particularly useful monomer having a hydrophilic group, the following monomers can be cited. For example, 2-hydroxyethyl acrylate, 2-hydroxyethyl methacrylate, glycerol monomethacrylate and the like hydroxyl group-containing monomers can be used particularly suitably. In addition, acrylic acid, methacrylic acid and the like carboxyl group-containing monomers or alkali metal salts and amine salts thereof can also be used suitably.

As another method for introducing a hydrophilic group into a solid phase of an organic material having no hydrophilic group, a material having a hydrophilic group can be coated. The material to be used in the coating is not particularly limited with the proviso that it has a hydrophilic group which is concerned in the adsorption of nucleic acid, but is preferably a polymer of an organic material from the viewpoint of easy handling. Examples of the polymer include polyhydroxyethyl acrylate, polyhydroxyethyl methacrylate and salts thereof, polyvinyl alcohol, polyvinyl pyrrolidone, polyacrylic acid, polymethacrylic acid and salts thereof, polyoxyethylene, acetyl cellulose, a mixture of acetyl celluloses having different acetyl values and the like, but a polymer having a polysaccharide structure is desirable.

Alternatively, it is possible to coat acetyl cellulose or a mixture of acetyl celluloses having different acetyl values on a solid phase of an organic material having no hydrophilic group and then to subject the coated acetyl cellulose or a mixture of acetyl celluloses having different acetyl values to a saponification treatment. In that case, the saponification ratio is preferably about 5% or more. The saponification ratio is more preferably 10% or more.

As the solid phase of an inorganic material having a hydrophilic group, a solid phase containing a silica compound can be exemplified. As the porous membrane containing a silica compound, a glass filter can be exemplified. Also can be exemplified is a porous silica thin membrane described in Japanese Patent No. 3,058,3442. This porous silica thin membrane can be prepared by spreading a developing solution of a cationic amphipathic substance having an ability to form a bimolecular membrane on a base material, preparing multi-layered bimolecular thin membranes of the amphipathic substance by removing the solvent from the liquid membrane on the base material, allowing the multi-layered bimolecular thin membranes to contact with a solution containing a silica compound, and then extracting and removing the aforementioned multi-layered bimolecular thin membranes.

Examples of the solid phase comprising inorganic material not having a hydrophilic group include aluminum and the like metals, glass, cement, pottery and the like ceramics, or a solid phase fabricated by stepping new ceramics, silicon, active charcoal, etc.

The method for introducing hydrophilic group into an inorganic material with no hydrophilic group includes the following two methods: one method comprises chemically binding an inorganic material to a graft polymer chain with hydrophilic group; and the other method comprises polymerizing a graft polymer chain starting from an inorganic material, using a monomer with hydrophilic group with double bonds within the molecule.

For chemical binding an inorganic material to a graft polymer chain with hydrophilic group, a functional group reactive with a functional group at the end of the graft polymer is introduced into an inorganic material, to which the graft polymer is chemically bound. For polymerizing a graft polymer chain starting from an inorganic material, using a monomer with hydrophilic group with double bonds within the molecule, a functional group as the start in polymerizing a compound with double bonds is introduced into an inorganic material.

As the graft polymer having a hydrophilic group and hydrophilic group-containing monomer having a double bond in the molecule, the aforementioned graft polymer having a hydrophilic group and hydrophilic group-containing monomer having a double bond in the molecule, described in the foregoing regarding the method for introducing a hydrophilic group into a solid phase of an organic material having no hydrophilic group, can be suitably use.

Another method for introducing a hydrophilic group to a solid phase comprising inorganic material not having a hydrophilic group is to coat a material having a hydrophilic group thereon. Materials used in coating are not limited as long as the hydroxyl group participates in the adsorption of nucleic acid, but for easy workability, a polymer of organic material is preferred. Examples of polymer include polyhydroxyethylacrylate, polyhydroxyethylmethacrylate and their salts, polyvinyl alcohol, polyvinylpyrrolidone, polyacrylate, polymethacrylate and their salts, polyoxyethylene, acetyl cellulose, and a mixture of acetyl celluloses which are different in acetyl value from each other.

To the solid phase comprising inorganic material not having a hydrophilic group, acetyl cellulose or a mixture of acetyl celluloses which are different in acetyl value from each other is coated thereon, and the coated acetyl cellulose and a mixture of acetyl celluloses which are different in acetyl value from each other can be saponified. In this case, the surface saponification degree in a range of 5% or more is preferred. It is more preferred to have the surface saponification degree in a range of 10% or more.

### {Properties}

The solid phase may be in any shape with which the solution can be in contact. The solid phase may be for example in a shape of which the surface is in contact with the solution, such as fiber and in a shape through which the solution can pass, as described below.

Additionally, the solid phase may comprise beads coated with the material described above. Such beads may preferably be coated with a mixture of acetylcellulose types with different acetyl values. In this case, magnetic beads may satisfactorily be used as the beads. For example, a triacetylcellulose membrane may satisfactorily be formed on the surface of polyethylene beads. In other words, triacetylcellulose is used for coating beads. Any material never causing contamination in nucleic acid or the like may be used for beads. Therefore, the material is not limited to polyethylene.

The solid phase is preferably used in a filter or membrane shape where the solution passes through the inside (sometimes referred to as solution-passable solid phase hereinbelow). In this case, the thickness is preferably 10 µm to 500 µm. More preferably, the thickness is 50 µm to 250 µm. The thickness within the range is preferable from the washing standpoint.

The solution-passable solid phase is preferably a porous membrane with a mean pore size of preferably 0.1 µm to 10 µm. More preferably, the mean pore size is 1 µm to 5 µm. Within the range, preferably, a surface area sufficiently enough to adsorb nucleic acid thereon can be obtained, with difficulty in clogging. The mean pore size of the solution-passable solid phase can be determined by the bubble point method (according to ASTM F316-86, JIS K-3832).

The solution-passable solid phase may be a porous membrane with symmetrical surface and back. Additionally, the solid phase may be a porous membrane with asymmetrical surface and back. Herein, the asymmetry of surface and back means that the physical property or chemical property of the porous membrane varies from one face thereof to the other face thereof.

Examples of the physical membrane property include mean pore size. Additionally, the chemical membrane property includes for example saponification degree.

In case that a porous membrane with asymmetric surface and back in terms of mean pore size is used in accordance with the invention, such porous membrane with a mean pore size varying in a decreasing manner along the direction of the flow of the solution is preferably used. Herein, a porous membrane at a 2 or more ratio of the maximum pore size and the minimum pore size is preferably used. More specifically, the ratio of the maximum pore size and the minimum pore size is 5 or more. Within the range, preferably, a surface area sufficiently enough to adsorb nucleic acid thereon can be obtained, with difficulty in clogging.

The nucleic acid-adsorbing porous membrane capable of passing a solution through the inside of the membrane having the percentage of porosity in a range of 50 to 95% is preferred. More preferable percentage of porosity is in a range of 65 to 80%. Further, having a bubble point in a range of 0.1 to 10 kgf/cm² is preferred. More preferable bubble point is in a range of 0.2 to 4 kgf/cm².

The nucleic acid-adsorbing porous membrane capable of passing a solution through the inside of the membrane having a pressure loss in a range of 0.1 to 100 kPa is preferred. As a result, a uniformed pressure can be obtained at pressurized states. More preferable pressure loss is in a range of 0.5 to 50 kPa. Herein, the term "pressure loss" represents the minimum pressure necessary for passing water through per 100 µm thickness of a membrane.

The nucleic acid-adsorbing porous membrane capable of passing a solution through the inside of the membrane having an amount of water percolation, at the time of passing water through under 1 kg/cm² pressure at 25°C, in a range of 1 to 5000 mL per 1 cm² membrane for 1 minute is preferred. More preferable amount of water percolation, at the time of passing water through under 1 kg/cm² pressure at 25°C, is in a range of 5 to 1000 mL per 1 cm² membrane for 1 minute.

The nucleic acid-adsorbing solid phase capable of passing a solution through the inside of the solid phase having an amount of nucleic acid-adsorption of 0.1 µg or more per 1 mg of a solid phase is preferred. More preferable amount of nucleic acid-adsorption is 0.9 µg or more per 1 mg of a porous membrane.

When passing a nucleic acid mixture solution through a nucleic acid-adsorbing solid phase, it is preferred to have the flow rate in a range of 2 to 1500 µL/sec per unit area cm² of the solid phase to obtain suitable contact time of the solution to the solid phase. When the contact time of the solution to the solid phase is too short, sufficient separation and purification effect cannot be obtained, and when too long, it is not preferred due to its operability. The flow rate in a range of 5 to 700µL/sec per unit area cm² of the solid phase is preferred.

In addition, the nucleic acid-adsorbing solid phase capable of passing a solution through the inside of the solid phase can be used in one layer, but also can be used in multi-layers. The multi-layers of the nucleic acid-adsorbing solid phase can be identical to or different from each other.

An illustration will now be made for a washing step as hereinafter. As a result of conducting a washing, recovered amount and purity of nucleic acid are enhanced and necessary amount of a test body containing nucleic acid is able to be made small. With regard to the washing step, one step will be acceptable for a purpose of quickening while, if purity is more important, it is preferred to repeat the washing for plural times.

The washing solution is preferably a solution containing a water-soluble organic solvent and/or a water-soluble salt. If necessary, satisfactorily, the washing solution may additionally contain buffers and surfactants. The washing solution is required to have a function to wash off impurities, adsorbed along with nucleic acid onto the solid phase and contained in a sample solution. Therefore, the washing solution should have a composition to desorb impurities from the solid phase without the desorption of nucleic acid therefrom. For that purpose, water-soluble organic solvents are suitable for allowing the components except nucleic acid to be desorbed therefrom while retaining nucleic acid thereon, because nucleic acid is slightly soluble in such water-soluble organic solvents. Additionally, the addition of water-soluble salts enables the elevation of the desorption effect of nucleic acid, so that selective elimination of impurities and unnecessary components can be enhanced, preferably.

With regard to a water-soluble organic solvent to be contained in a washing solution, methanol, ethanol, isopropanol, n-propanol, butanol, acetone, etc, may be used and, among them, it is preferred to use ethanol. Amount of the water-soluble organic solvent contained in the washing solution is preferably 20 to 100% by weight and, more preferably, 40 to 80% by weight.

On the other hand, for the water-soluble salt contained in a washing solution, a halide salt is preferred and among them, a chloride salt is more preferred. Further, the water-soluble salt is preferably a monovalent or divalent cation, particularly an alkali metal and an alkali earth metal is preferred. And among them, a sodium salt and a potassium salt are most preferred, and a sodium salt is particularly preferred. When the water-soluble salt is contained in the washing solution, the concentration thereof is preferable 10 mmol/L or more, and the upper limit is not particularly limited as long as the upper limit does not affect solubility of the impurities, 1 mol/L or less is preferred and 0.1 mol/L or less is more preferred. Above all, that the water-soluble salt is sodium chloride and sodium chloride is contained in 20 mmol/L or more is particularly preferred.

The buffers and surfactants include the buffers and surfactants already described above in the section {Pretreating solution}. Among them, the washing solution preferably contains ethanol, Tris and Triton X-100. The preferable concentrations of Tris and Triton X-100 are 10 to 100 mmol/L and 0.1 to 10 % by mass, respectively.

In addition, the washing solution is characterized in that a chaotropic substance is not contained therein. As a result, a possibility of having the chaotropic substance incorporated into a recovery step after the washing step can be reduced. In the recovery step, where the chaotropic substance is incorporated thereinto, it sometimes hinders an enzyme reaction such a PCR reaction or the like, therefore considering the afterward enzyme reaction, not including the chaotropic substance to a washing solution is ideal. Further, the chaotropic substance is corrosive and harmful, in this regard, it is extremely advantageous from an operational safety standpoint for the researcher not to use the chaotropic substance when unnecessary.

Herein, the chaotropic substance represents aforementioned urea, guanidine chloride, guanidine isothiocyanate, guanidine thiocyanate, sodium isothiocyanate, sodium iodide, potassium iodide, etc.

Since the washing solution has high wettability for a container, the washing solution sometimes remains in the container during the washing step in the nucleic acid separation purification process, so that the recovery step after the washing step is contaminated with the washing solution to cause reduction of the purity of nucleic acid and reduction of the reactivity in the subsequent step. Thus, in the first method and the second method of the present invention, when adsorption and desorption of nucleic acid are carried out using a container, it is important that a solution to be used in the adsorption or washing, particularly the washing solution, does not remain in the cartridge so that it does not exert influence upon the next step.

Accordingly, in order to prevent contamination of the elution solution of the subsequent step with the washing solution of the washing step and thereby to keep residue of the washing solution in the cartridge to the minimum, it is desirable that surface tension of the washing solution is less than 0.035 J/m². When the surface tension is low, wettability of the washing solution for the cartridge is improved and volume of the residual solution can be controlled.

The washing efficiency of the washing solution can be elevated by elevating the ratio of water. But the surface tension of the resulting washing solution is also increased in that case, so that the volume of the remaining liquid is increased. In case that the surface tension of the washing solution is 0.035 J/m² or more, the volume of the remaining liquid can be reduced by elevating the repellency of the container. By elevating the repellency of the container, liquid droplets are formed. When the liquid droplets flow down, the volume of the remaining liquid can be reduced. The method for elevating repellency includes for example but is not limited to a process of coating repellents such as silicone on the surface of the container or a process of kneading repellents such as silicone during the molding process of the container.

In a washing procedure, the amount of a washing solution is preferably 2 µl/mm² or more. When large quantity of the washing solution is used, the washing effect could improve, but in order to maintain the operationability and prohibit the sample from discharging, 200 µl/mm² or less is preferred.

In a washing procedure, when passing a washing solution through a nucleic acid-adsorbing porous membrane, it is preferred to have the flow rate in a range of 2 to 1500 µl/sec per unit area (cm²) of the membrane, and more preferably in a range of 5 to 700 µl/sec. Normally, the passing speed is reduced to elongate the time so that washing is sufficiently conducted. However, preferably, by using the aforementioned range in the invention the step for separating and purifying nucleic acid can be conducted rapidly without reducing the washing efficiency.

In the washing step, a temperature of the washing solution in a range of 4 to 70°C: is preferred. Further, a temperature of the washing solution at room temperature is more preferred. In addition to the washing step, stirring using an ultrasonic or a mechanical vibration can be applied to the cartridge for separation and purification of nucleic acid at the same time. On the other hand, washing can be done by conducting a centrifugation.

### <Recovering process (desorption process)>

Following the washing step, the solid phase after washing is put in contact with a elution solution as the solution capable of desorbing nucleic acid adsorbed to the solid phase. Because the solution resulting from the contact with the solid phase (sometimes referred to as post-purification solution hereinafter) contains the intended nucleic acid, the solution is subjected to the following step, for example PCR (polymerase chain reaction) amplification of nucleic acid.

Volume of a elution solution to volume of the sample solution containing nucleic acid prepared from a test body is adjusted whereby desorption of nucleic acid is able to be carried out. Volume of the recovered solution containing nucleic acid which is separated and purified is dependent upon the amount of the test body used at that time. Although the commonly used amount of the recovered solution is from several tens to several hundred µl, that may be changed within a range of 1 µl to several tens ml when the sample amount is very little or, reversely, a large amount of nucleic acid is to be separated and purified.

For the elution solution, purified distilled water, Tris/EDTA buffer and the like can preferably be used.

It is preferred that pH of a elution solution is 2 to 11 and, more preferably, 5 to 9. In addition, ionic strength and salt concentration particularly affect the elution of adsorbed nucleic acid. Preferably, the elution solution has an ionic strength of 290 mmol/L or less and has a salt concentration of 90 mmol/L or less. As a result thereof, recovering rate of nucleic acid increases and much more nucleic acid is able to be recovered.

When volume of a elution solution is made small as compared with the initial volume of a sample solution containing nucleic acid, it is now possible to prepare a recovered solution containing concentrated nucleic acid. Preferably, the ratio of (volume of elution solution), (volume of sample solution) is able to be made 1.100 to 99:100 and, more preferably, it is able to be made 1:10 to 9:10. As a result thereof, nucleic acid is now able to be easily concentrated without conducting an operation for concentrating in a step after separation and purification of nucleic acid. According to such a method, a method for producing a nucleic acid solution in which nucleic acid is concentrated as compared with a test body is able to be provided.

There is no limitation for the infusing times for a elution solution and that may be either once or plural times. Usually, when nucleic acid is to be separated and purified quickly and simply, that is carried out by means of one recovery while, when a large amount of nucleic acid is to be recovered, elution solution may be infused for several times.

Also, in the recovering step, it is possible to add a stabilizing agent for preventing degradation of nucleic acid recovered in the elution solution of nucleic acid. As the stabilizing agent, an antibacterial agent, a fungicide, a nucleic acid degradation inhibitor and the like can be added. As the nuclease inhibitor, ETSTA and the like can be cited. In addition, as another embodiment, a stabilizer can also be added to the recovery container in advance.

### <Cartridge for separating and purifying nucleic acid>

According to the method for separating and purifying nucleic acid in accordance with the invention, preferably, a cartridge for separating and purifying nucleic acid can be used for carrying out the adsorption and desorption of nucleic acid, where the solid phase is placed inside a container with at least two openings.

The material of the container is not specifically limited as long as the container can hold the solid phase and at least two openings can be arranged in the container. From the respect of ready production, plastics are preferable. Preferably, transparent or opaque plastics for example polystyrene, polymethacrylate ester, polyethylene, polypropylene, polyester, nylon, and polycarbonate are used.

The shape of the solid phase placed in the container is not specifically limited. An appropriate shape may be satisfactory, including circle, square, rectangle, and oblique; cylinder-shaped membrane and roll-shaped membrane; or beads coated with an organic polymer with hydroxyl group on the surface. From the suitability in production, highly symmetrical shapes such as circle, square, cylinder, and roll or beads are preferable.

The inner volume of the container is preferably determined, on the basis of the volume of the sample solution to be treated. Generally, the inner volume is expressed as the volume of the solid phase placed therein. Specifically, the inner volume is preferably at a dimension capable of holding about one to six sheets of a solid phase of a thickness of about 1 mm or less (e.g., about 50 to 500 µm) and a diameter of about 2 mm to 20 mm.

The end face of the solid phase in contact with the container preferably adheres closely to the inner wall face of the container at such a level that the sample solution or the like never passes through a space if any.

In a view of the side of the solid phase, the face of the solid phase on the side of an opening to be used as the inlet of a sample solution and the like (on the opening side from the solid phase in the container) in the container with at least two openings preferably never adheres closely to the inner wall of the container but retains a space from the inner wall thereof to make a structure where the sample solution and the like are dispersed as uniformly as possible on the whole surface of the solid phase.

### <Unit for separating and purifying nucleic acid>

More preferably, a unit for separating and purifying nucleic acid is used for the method for separating and purifying nucleic acid in accordance with the invention, the unit comprising
(a) a solid phase,
(b) a container with at least two openings, for placing the solid phase therein, and
(c) a pressure difference-generating apparatus connected to one of the openings of the container.

Because those described in (a) and (b) in the unit for separating and purifying nucleic acid are the same as the cartridge for separating and purifying nucleic acid, the parts in the unit for separating and purifying nucleic acid are sometimes referred to as cartridge for separating and purifying nucleic acid. The unit for separating and purifying nucleic acid is described below.

The container satisfactorily has a part for placing the solid phase therein to hold the solid phase in the part, where the solid phase never goes out of the part during the aspiration and discharge of a sample solution and the like. Satisfactorily, a pressure difference-generating apparatus can be connected to the opening. Therefore, the container is initially divided in two parts, which are preferably integrated together after the solid phase is placed in the container. Additionally, a mesh prepared of a material never causing contamination in nucleic acid can be arranged above and under the solid phase, by which it can be avoided for the solid phase to go out of the part for placing the solid phase therein.

The container is generally prepared in an embodiment such that a body for placing the solid phase and a lid are separately arranged. At least one opening is arranged in any of the two. The openings are used as an inlet and an outlet for a sample solution containing nucleic acid, the washing solution and the elution solution (referred to as "sample solution, etc." hereinbelow) and are connected to a pressure difference-generating apparatus allowing the inside of the container to be under reduced pressure or at a pressurized state. The shape of the body is not specifically limited. For ready production and ready dispersion of the sample solution, etc. over the whole surface of the solid phase, the cross section of the body is preferably circular. A cross section of square is also preferred so as to prevent the generation of cut pieces in producing the solid phase.

The lid is essentially connected to the body so as to allow the inside of the container to be under reduced pressure or at a pressurized state with a pressure difference-generating apparatus. As long as that state can be successfully realized, any connecting method is appropriately selected.

The connecting method includes for example the use of adhesives, screwing, engagement, screw fastening, and fusion with ultrasonic wave.

The pressure difference-generating apparatus includes syringe, pipette, or pumps capable of aspiration and pressurization such as perista pump. Among them, syringe is suitable for manual procedure, while pumps are suitable for automatic procedure. Additionally, pipette has an advantage such that pipette can be readily manipulated with a single one hand.

Preferably, the pressure difference-generating apparatus is connected in a removable manner to one of the openings of the container.

When three or more openings are arranged on or in the container, further, it is needless to say that extra-openings should be temporarily closed so as to enable liquid aspiration and discharge, following the procedures under reduced pressure or at a pressurized state.

A first mode of the method for separating and purifying nucleic acid in accordance with the invention includes the following steps.
(1a) A step of preparing a sample solution containing nucleic acid, using a sample and inserting one of the openings of a container with at least two openings for placing a solid phase therein into the resulting sample solution.
(1b) A step of permitting the inside of the container under reduced pressure, using a pressure difference-generating apparatus connected to the other opening of the container with at least two openings for placing the solid phase therein, to aspirate the sample solution containing nucleic acid and put the sample solution in contact with the solid phase.
(1c) A step of permitting the inside of the container at a pressurized state using the pressure difference-generating apparatus connected to the other opening, to discharge the aspirated sample solution containing nucleic acid outside the container.
(1d) A step of inserting the one of the openings into a washing solution.
(1e) A step of permitting the inside of the container under reduced pressure, using the pressure difference-generating apparatus connected to the other opening, to aspirate the washing solution and put the washing solution in contact with the solid phase.
(1f) A step of permitting the inside of the container at a pressurized state using the pressure difference-generating apparatus connected to the other opening, to discharge the aspirated washing solution outside the container.
(1g) A step of inserting the one of the openings into a elution solution.
(1h) A step of permitting the inside of the container under reduced pressure, using the pressure difference-generating apparatus connected to the other opening, to aspirate the elution solution and put the elution solution in contact with the solid phase.
(1i) A step of permitting the inside of the container at a pressurized state using the pressure difference-generating apparatus connected to the other opening, to discharge the elution solution outside the container.

At the (1b), (1e) and (1h), the solutions at a volume capable of the contact with nearly the whole solid phase are preferably aspirated. Because the pressure difference-generating apparatus may be contaminated when aspiration is done into the inside of the apparatus, however, the volume should be adjusted to an appropriate volume. After aspirating an appropriate volume of the solutions, the inside of the container is pressurized using the pressure difference-generating apparatus, to then discharge the aspirated solutions. No interval is needed up to these procedures. Immediately after aspiration, discharge may satisfactorily be done.

Preferable one embodiment of the unit for separating and purifying nucleic acid for practicing the first mode includes for example the apparatus for separating and purifying nucleic acid as described in Japanese Laid-Open No. 2004/180637.

In the unit for separating and purifying nucleic acid for carrying out the first mode, a member with a hole nearly at its center is preferably arranged on the solid phase facing the opening connected to the pressure difference-generating apparatus.

The member has a function for pressing down the solid phase and also has an effect on efficient discharge of the sample solution, etc. So as to draw the liquid in the center hole, the member is preferably in a shape with a slant face, such as funnel or bowl. A person skilled in the art can appropriately determine the size of the hole, the angle of the slant face, and the thickness of the member, taking account of the volume of the sample solution, etc., the size of the container for placing the solid phase therein, and the like. In between the member and the opening, there is preferably arranged a space for reserving the overflow of the sample solution, etc. to prevent the aspiration thereof into the pressure difference-generating apparatus. The size of the space can appropriately be selected by a person skilled in the art. So as to efficiently collect nucleic acid, a sample solution containing nucleic acid at a volume enough for the impregnation of the whole solid phase or a volume larger than that is preferably aspirated.

So as to prevent the centralization of the sample solution, etc. only directly below the opening during aspiration to allow the sample solution, etc. to pass through the solid phase relatively uniformly, a space is preferably arranged in between the solid phase and the member. For that purpose, plural protrusions are preferably arranged on the member toward the solid phase. The size and number of the protrusions can appropriately be selected by a person skilled in the art. The opening area of the solid phase is preferably retained as large as possible, as long as the space is still retained.

A second mode of the method for separating and purifying nucleic acid in accordance with the invention comprises the following steps.
(2a) A step of preparing a sample solution containing nucleic acid using a sample and injecting the sample solution containing nucleic acid into one of the openings of the container with at least two openings for placing a solid phase therein.
(2b) A step of connecting a pressure difference-generating apparatus to the one of the openings to make the inside of the container at a pressurized state, and discharging the injected sample solution containing nucleic acid from the other opening of the container with at least two openings for placing the solid phase therein, to put the sample solution in contact with the solid phase.
(2c) A step of removing the pressure difference-generating apparatus from the one of the openings and injecting a washing solution into the one of the openings of the cartridge for separating and purifying nucleic acid.
(2d) A step of connecting a pressure difference-generating apparatus to the one of the openings to make the inside of the container at a pressurized state, and discharging the injected washing solution from the other opening, to put the washing solution in contact with the solid phase.
(2e) A step of removing the pressure difference-generating apparatus from the one of the openings and injecting a elution solution into the one of the openings of the cartridge for separating and purifying nucleic acid.
(2f) A step of connecting the pressure difference-generating apparatus to the one of the openings to make the inside of the container at a pressurized state, and discharging the injected elution solution from the other opening, to allow the nucleic acid adsorbed to the solid phase to be desorbed from the solid phase and then discharge the nucleic acid.

At the step, the method for adding the sample solution to the container is not limited. Experimental tools such as pipette and syringe are preferably used. These tools are more preferably nuclease-free or pyrogen-free.

One mode for carrying out the method for separating and purifying nucleic acid in accordance with the invention may be done using an automatic apparatus, with no limitation. For example, the automatic apparatus hereinbelow described is exemplified with no specific limitation.

The automatic apparatus is an apparatus for separating and purifying nucleic acid for automatic separation and purification using a container with at least two openings for preliminarily placing a solid phase capable of adsorbing nucleic acid therein, where a solution can pass through the inside (cartridge for separating and purifying nucleic acid). The automatic apparatus automatically carries out separation and purification procedures including the steps of injecting a sample solution containing nucleic acid into the cartridge for separating and purifying nucleic acid, pressurizing the cartridge to allow the nucleic acid in the sample solution to be adsorbed to the solid phase, charging a washing solution in a dividend manner in the cartridge for separating and purifying nucleic acid and pressurizing the cartridge to remove impurities, subsequently charging a elution solution in a dividend manner in the cartridge for separating and purifying nucleic acid to allow the nucleic acid adsorbed to the solid phase to be desorbed from the solid phase to recover the nucleic acid together with the elution solution. The automatic apparatus preferably comprises a mounting mechanism for holding the cartridge for separating and purifying nucleic acid, a liquid waste container for placing therein the discharged solutions of the sample solution and the washing solution, and a recovering container for placing the elution solution containing nucleic acid therein; a pressurized air supply mechanism for introducing pressurized air into the cartridge for separating and purifying nucleic acid; and a dividend injection mechanism for charging the washing solution and the elution solution in a dividend manner in the cartridge for separating and purifying nucleic acid.

As described above, the automatic apparatus is equipped with a mounting mechanism for holding the cartridge for separating and purifying nucleic acid, a liquid waste container and a recovering container; a pressurized air supply mechanism for introducing pressurized air into the cartridge for separating and purifying nucleic acid; and a dividend injection mechanism for charging the washing solution and the elution solution in a dividend manner in the cartridge for separating and purifying nucleic acid. The automatic apparatus automatically carries out the steps for separating and purifying nucleic acid, including a step of injecting a sample solution containing nucleic acid into the cartridge for separating and purifying nucleic acid, for pressurization to allow the nucleic acid to be adsorbed to the solid phase member, a step of injecting a washing solution in a dividend manner to wash off impurities, a step of injecting a elution solution in a dividend manner to separate and recover the nucleic acid adsorbed to the solid phase member. Such automatic apparatus can be prepared into a compact constitution with a mechanism for automatically separating and purifying nucleic acid in a sample solution, highly efficiently in a short time.

The invention is now described in detail in the following Examples. The invention is never limited to them.

### [Example 1]

### (1) Preparation of container with at least two openings

A container of an inner diameter of 7 mm and with at least two openings and a part for placing a porous membrane capable of adsorbing nucleic acid thereon as a solid phase was prepared of high-impact polystyrene.

### (2) Preparation of cartridge for separating and purifying nucleic acid

As the porous membrane capable of adsorbing nucleic acid, a porous membrane (pore diameter of 2.5 µm, diameter of 7 mm, thickness of 100 µm, and saponification ratio at 95 %) prepared by saponification process of triacetylcellulose porous membrane was used and placed in the part for placing therein a porous membrane capable of adsorbing nucleic acid in the container with at least two openings, as prepared above in (1), to prepare a cartridge for separating and purifying nucleic acid.

### (3) Preparation of pretreating solution and washing solution

The pretreating solution and the washing solution with the formulas described below were prepared.

### (Pretreating solution)

| | |
|---|---|
| Guanidine hydrochloride (manufactured by Wako Pure Chemicals Industries, Ltd. | 946 g |
| CTAB {cetyltrimethyl ammonium bromide (manufactured by Wako Pure Chemicals Industries, Ltd.)} | 40 g |
| Ethanol | 116 g |
| Leodol TWS-120V (manufactured by Kao) | 59 g |
| AK-02 (manufactured by Shin-etsu Chemical) | 10 g |
| Distilled water | 1030 g |

### (Washing solution)

10 mM Tris-HCl (manufactured by Nippon Gene) 50 % ethanol

### (4) Procedures for separating and purifying nucleic acid

250 µl of the pretreating solution was added to 30 µl of a proteinase (Proteinase K; manufactured by Merck) and 200 µl of human whole blood in this order. The resulting solution was mixed together under conditions in Table 1. Subsequently, the mixture solution was incubated at 60 °C for 10 minutes. After incubation, 250 µl of 100 % by volume of ethanol was added for vortexing at 2500 rpm for 15 seconds. Subsequently, the resulting mixture solution was injected into one of the openings in the cartridge equipped with the nucleic acid-ad sorptive porous membrane prepared above in (2) for separating and purifying nucleic acid. A pressure difference-generating apparatus (tubing pump) was successively connected to the one of the openings. By making the inside of the cartridge for separating and purifying nucleic acid at a pressurized state (80 kpa) and then passing the injected sample solution containing nucleic acid through the nucleic acid-adsorptive porous membrane, the sample solution was put in contact with the nucleic acid-adsorptive porous membrane and then discharged from the other opening of the cartridge for separating and purifying nucleic acid. Continuously, the pressure difference-generating apparatus was removed from the one of the openings. Then, 700 µl of the washing solution was injected into the one of the openings of the cartridge for separating and purifying nucleic acid. The tubing pump was connected to the one of the openings, to make the inside of the cartridge for separating and purifying nucleic acid at a pressurized state (80 kpa), and then, the injected washing solution was passed through the nucleic acid-adsorptive porous membrane and discharged from the other opening. Continuously, the pressure difference-generating apparatus was removed from the one of the openings. Then, a elution solution (200 µl of distilled water) was injected into the one of the openings of the cartridge for separating and purifying nucleic acid. The tubing pump was connected to the one of the openings, to make the inside of the cartridge for separating and purifying nucleic acid at a pressurized state (80 kpa), and then, the injected elution solution was passed through the nucleic acid-adsorptive porous membrane and discharged from the other opening, which was recovered. The series of procedures were done at ambient temperature.

**[Table 1]**

| Sample No. | Mixing Process | DNA (µg) | |
|---|---|---|---|
| 1 | Vortexing 2500rpm 15sec. | 4.8 | Invention |
| 2 | Vortexing 1800rpm 15sec. | 3.3 | Comparison |
| 3 | Mixing with inversion: five times + Vortexing 1800rpm 15sec. | 5.0 | Invention Invention |
| 4 | Pipetting: five times + Vortexing 1800rpm 15sec. | 4.5 | Invention |

As apparently shown in the results of Table 1, it is indicated that vortexing at 2500 rpm or more or a combination mixing with inversion or pipetting with vortexing can increase the amount of DNA recovered.

### [Example 2]

### (1) Preparation of container with at least two openings

A container of an inner diameter of 7 mm and with at least two openings and a part for placing a porous membrane capable of adsorbing nucleic acid as a solid phase was prepared of high-impact polystyrene.

### (2) Preparation of cartridge for separating and purifying nucleic acid

As the porous membrane capable of adsorbing nucleic acid, a porous membrane (pore diameter of 2.5 µm, diameter of 7 mm, thickness of 100 µm, and saponification ratio at 95 %) prepared by saponification process of triacetyl cellulose porous membrane was used and placed in the part for placing a porous membrane capable of adsorbing nucleic acid in the container with at least two openings as prepared above in (1), to prepare a cartridge for separating and purifying nucleic acid.

### (3) Preparation of pretreating solution and washing solution

The pretreating solution and the washing solution with the formulas in Example 1, (3) were prepared.

### (4) Procedures for separating and purifying nucleic acid

250 µl of the pretreating solution was added to 30 µl of a proteinase (Proteinase K; manufactured by Merck) and 200 µl of human whole blood in this order, for vortexing at 2500 rpm for 15 seconds. Subsequently, the mixture solution was incubated at 60 °C for 10 minutes. After incubation, 250 µl of 100 % by volume of ethanol was added, and the resulting solution was mixed together under the conditions in Table 2. Subsequently, the resulting mixture solution was injected into one of the openings in the cartridge for separating and purifying nucleic acid, as prepared above in (2). A pressure difference-generating apparatus (tubing pump) was successively connected to the one of the openings. By making the inside of the cartridge for separating and purifying nucleic acid at a pressurized state (80 kpa) and then passing the injected sample solution containing nucleic acid through the nucleic acid-adsorptive porous membrane, the sample solution was put in contact with the nucleic acid-adsorptive porous membrane and then discharged from the other opening of the cartridge for separating and purifying nucleic acid. Continuously, the pressure difference-generating apparatus was removed from the one of the openings. Then, 700 µl of the washing solution was injected into the one of the openings of the cartridge for separating and purifying nucleic acid. The tubing pump was connected to the one of the openings, to make the inside of the cartridge for separating and purifying nucleic acid at a pressurized state (80 kpa), and then, the injected washing solution was passed through the nucleic acid-adsorptive porous membrane and then discharged from the other opening. Continuously, the pressure difference-generating apparatus was removed from the one of the openings. Then, a elution solution (200 µl of distilled water) was injected into the one of the openings of the cartridge for separating and purifying nucleic acid. The tubing pump was connected to the one of the openings, to make the inside of the cartridge for separating and purifying nucleic acid at a pressurized state (80 kpa), and then, the injected elution solution was passed through the nucleic acid-adsorptive porous membrane and then discharged from the other opening, which was recovered. The series of procedures was done at ambient temperature.

**[Table 2]**

| Sample No. | Mixing Process | DNA (*µ* g) | |
|---|---|---|---|
| 1 | Vortexing 2500rpm 15sec. | 4.5 | Invention |
| 2 | Vortexing 1800rpm 15sec. | 3.1 | Comparison |
| 3 | Mixing with inversion: five times + Vortexing 1800rpm 15sec. | 4.2 | Invention |
| 4 | Pipetting: five times + Vortexing 1800rpm 15sec. | 4.2 | Invention |

As apparently shown in the results of Table 2, it is indicated that vortexing at 2500 rpm or more or a combination mixing with inversion or pipetting with vortexing can increase the amount of DNA recovered.

### [Example 3]

### (1) Preparation of container having at least two openings

A container having at least two openings of an inner diameter of 7 mm and with a part for placing nucleic acid-adsorptive porous membrane therein was prepared of high-impact polystyrene.

### (2) Preparation of cartridge for separating and purifying nucleic acid

As the nucleic acid-adsorptive porous membrane, a porous membrane (pore diameter of 2.5 µm, diameter of 7 mm, thickness of 100 µm, and saponification ratio at 95 %) prepared by saponification process of triacetylcellulose porous membrane is used and placed in the part for placing the nucleic acid-adsorptive porous membrane in the container having at least two openings as prepared in (1) to prepare a cartridge for separating and purifying nucleic acid.

### (3) Preparation of a pretreating solution and a washing solution

A pretreating solution and a washing solution with the formulas as set forth below, respectively were prepared.

### Pretreating solution (adsorption buffer solution for purifying nucleic acid) (the invention)

| | |
|---|---|
| Guanidine hydrochloride (manufactured by Wako Pure Chemicals Industries, Ltd.) | 946 g |
| CTAB (cetyltrimethyl ammonium bromide; manufactured by Wako Pure Chemicals Industries, Ltd.) | 40 g |
| Ethanol | 116 g |
| Leodol TWS-120V (manufactured by Kao) | 59 g |
| AK-02 (manufactured by Shin-etsu Chemical) | 10 g |
| Distilled water | 1030 g |

### Washing solution (buffer for washing nucleic acid)

| | |
|---|---|
| 10 mM Tris-HCl (manufactured by Nippon Gene) | 50 % ethanol |

### (4) Procedures for separating and purifying nucleic acid

250 µl of the pretreating solution was added to 30 µl of a proteinase (Proteinase K; manufactured by Merck) and 200 µl of human whole blood, in this order. The resulting solution was then mixed together under conditions described in Table 3 below. Subsequently, the mixture solution was incubated at 60 °C for 10 minutes. After incubation, 250 µl of 100 % by volume of ethanol was added for agitation with vortexing at 2500 rpm, for 15 seconds. After agitation, the resulting mixture solution was injected into one of the openings in the cartridge for separating and purifying nucleic acid being equipped with the nucleic acid-adsorptive porous membrane prepared above in (2). A pressure difference-generating apparatus (tubing pump) was successively connected to the one of the openings. By subsequently making the inside of the cartridge for separating and purifying nucleic acid at a pressurized state (80 kpa) and then passing the injected sample solution containing nucleic acid through the nucleic acid-adsorptive porous membrane, the sample solution was put in contact with the nucleic acid-adsorptive porous membrane and then discharged from the other opening of the cartridge for separating and purifying nucleic acid. Continuously, 700 µl of the washing solution was injected into the one of the openings of the cartridge for separating and purifying nucleic acid. The tubing pump was connected to the one of the openings, to make the inside of the cartridge for separating and purifying nucleic acid at a pressurized state (80 kpa), and then, the injected washing solution was passed through the nucleic acid-adsorptive porous membrane and then discharged from the other opening. Continuously, a elution solution (200 µl of distilled water) was injected into the one of the openings of the cartridge for separating and purifying nucleic acid. The tubing pump was connected to the one of the openings, to make the inside of the cartridge for separating and purifying nucleic acid at a pressurized state (80 kpa), and then, the injected elution solution was passed through the nucleic acid-adsorptive porous membrane and then discharged from the other opening, which was recovered. The series of procedures was done at ambient temperature.

The series of the procedures in each sample was done. The results are shown in Table 3.

**Table 3**

| No. | Mixing method | DNA (µg) |
|---|---|---|
| 1. | 2500 rpm for 15 sec. | 4.6 |
| 2. | 2300 rpm for 15 sec. | 4.7 |
| 3. | 2000 rpm for 15 sec. | 4.7 |
| 4. | 1800 rpm for 15 sec. | 2.4 |
| 5. | 1600 rpm for 15 sec. | 1.7 |

As seen from the results in Table 3, the agitation at 2000 rpm or more leads to recovery of a sufficient amount of DNA. As seen from the results in Table 3, and Tables 1 and 2, in case of the agitation at less than 2000 rpm, a combination of mixing with inversion or pipetting leads to the recovery of a sufficient amount of DNA.

This application is based on Japanese patent applications JP 2004-257202, filed on September 3, 2004 and JP 2005-253576, filed on September 1, 2005, the entire content of which is hereby incorporated by reference, the same as if set forth at length.

## Claims

1. A method for separating and purifying nucleic acid, comprising:
preparing a sample solution containing nucleic acid;
putting the sample solution containing nucleic acid in contact with a solid phase to allow nucleic acid to be adsorbed to the solid phase;
putting a washing solution in contact with the solid phase to wash the solid phase at the state of nucleic acid adsorbed thereon; and
putting a elution solution in contact with the solid phase to allow nucleic acid to be desorbed from the solid phase.
Wherein the step of preparing a sample solution containing nucleic acid comprises at least one selected from the group consisting of vortexing, mixing with inversion, and pipetting.

2. A method for separating and purifying nucleic acid according to claim 1, wherein the step of preparing a sample solution containing nucleic acid comprises: adding a proteinase, a sample, and a pretreating solution containing at least one selected from the group consisting of chaotropic salts, surfactants, defoaming agents, nucleic acid stabilizers and buffers, in this order, or adding the pretreating solution, a sample and a proteinase, in this order, and subsequently carrying out at least one selected from the group consisting of vortexing, mixing with inversion, and pipetting.

3. A method for separating and purifying nucleic acid according to claim 1, wherein the step of preparing a sample solution containing nucleic acid comprises: adding a proteinase, a sample, and a pretreating solution containing at least one selected from the group consisting of chaotropic salts, surfactants, defoaming agents, nucleic acid stabilizers and buffers, in this order, or adding the pretreating solution, a sample and a proteinase, in this order; adding a water-soluble organic solvent; and carrying out at least one selected from the group consisting of vortexing, mixing with inversion, and pipetting.

4. A method for separating and purifying nucleic acid according to claim 1, wherein the step of preparing a sample solution containing nucleic acid comprises: adding a proteinase, a sample, and a pretreating solution containing at least one selected from the group consisting of chaotropic salts, surfactants, defoaming agents, nucleic acid stabilizers and buffers, in this order, or adding the pretreating solution, a sample and a proteinase, in this order; carrying out at least one selected from the group consisting of vortexing, mixing with inversion, and pipetting; and adding a water-soluble organic solvent.

5. A method for separating and purifying nucleic acid according to claim 1, wherein the step of preparing a sample solution containing nucleic acid comprises: adding a proteinase, a sample, and a pretreating solution containing at least one selected from the group consisting of chaotropic salts, surfactants, defoaming agents, nucleic acid stabilizers and buffers, in this order, or adding the pretreating solution, a sample and a proteinase, in this order; subsequently carrying out at least one selected from the group consisting of vortexing, mixing with inversion, and pipetting; adding a water-soluble organic solvent; and carrying out at least one selected from the group consisting ofvortexing, mixing with inversion, and pipetting.

6. A method for separating and purifying nucleic acid according to claim 1, wherein the sample solution containing nucleic acid is obtained by the preparation of whole blood as a sample.

7. A method for separating and purifying nucleic acid according to claim 1, wherein the step of preparing a sample solution containing nucleic acid comprises vortexing at 2,000 rpm or more.

8. A method for separating and purifying nucleic acid according to claim 1, wherein the step of preparing a sample solution containing nucleic acid comprises performing once or more ot mixing with inversion or pipetting in combination with vortexing at less than 2,500 rpm.

9. A method for separating and purifying nucleic acid according to claim 1, wherein the step of preparing a sample solution containing nucleic acid comprises performing once or more of mixing with inversion or pipetting in combination with vortexing at less than 2,000 rpm.

10. A method for separating and purifying nucleic acid according to any one of claims 3 to 5, wherein the water-soluble organic solvent includes at least one selected from the group consisting of methanol, ethanol, propanol and butanol.

11. A method for separating and purifying nucleic acid according to claim 1, wherein the solid phase is in a membrane form.

12. A method for separating and purifying nucleic acid according to claim 1, wherein the solid phase contains silica or a derivative thereof, diatomaceous earth, or alumina.

13. A method for separating and purifying nucleic acid according to claim 1, wherein the solid phase contains an organic polymer.

14. A method for separating and purifying nucleic acid according to claim 13, wherein the organic polymer is an organic polymer having a polysaccharide structure.

15. A method for separating and purifying nucleic acid according to claim 13, wherein the organic polymer is acetylcellulose.

16. A method for separating and purifying nucleic acid according to claim 13, wherein the organic polymer is an organic polymer prepared by a process of saponifying acetylcellulose or a mixture of acetylcellulose having different acetyl values.

17. A method for separating and purifying nucleic acid according to claim 13, wherein the organic polymer is regenerated cellulose.
